(19) Europäisches Patentamt
European Patent Office
Office européen des brevets

(11) Veröffentlichungsnummer: **0 194 490**
**A2**

(12) # EUROPÄISCHE PATENTANMELDUNG

(21) Anmeldenummer: **86102309.1**

(22) Anmeldetag: **22.02.86**

(51) Int. Cl.[4]: **C 07 C 67/343**
C 07 C 69/738, C 07 C 79/46
C 07 C 76/02

(30) Priorität: **09.03.85 DE 3508533**

(43) Veröffentlichungstag der Anmeldung:
**17.09.86 Patentblatt 86/38**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Preiss, Michael, Dr.**
**Egenstrasse 21**
**D-5600 Wuppertal 1(DE)**

(54) **Verfahren zur Herstellung von Benzylidenverbindungen.**

(57) Die vorliegende Erfindung betrifft ein neues Verfahren zur Herstellung von substituierten Benzylidenverbindungen der allgemeinen Formel I

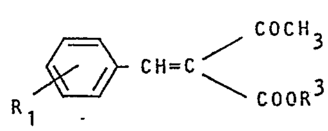

(I)

in welcher $R^1$ und $R^3$ die in der Beschreibung angegebene Bedeutung haben, aus aromatischen Acetalen und ß-Ketocarbonsäureestern sowie ihre Verwendung als Zwischenprodukte bei der Herstellung von biologisch aktiven Wirkstoffen.

EP 0 194 490 A2

BAYER AKTIENGESELLSCHAFT 5090 Leverkusen, Bayerwerk
Konzernverwaltung RP
Patentabteilung KS/by-c
IV(Pha)

## Verfahren zur Herstellung von Benzylidenverbindungen

Die vorliegende Erfindung betrifft ein neues Verfahren zur Herstellung von substituierten Benzylidenverbindungen aus aromatischen Acetalen und ß-Ketocarbonsäureestern sowie ihre Verwendung als Zwischenprodukte bei der Herstellung von biologisch aktiven Wirkstoffen.

Benzylidenverbindungen, ihre Herstellung und ihre Verwendung bei der Synthese von pharmazeutisch wirksamen Dihydropyridinen sind bereits bekannt (vgl. DE-OS 21 17 571 und DE-OS 21 17 573). Die bekannten Herstellungsverfahren beruhen im wesentlichen auf einer Kondensation von Aldehyden mit Acetessigsäureestern. Die Verwendung von Aldehyden ist in vielen Fällen unerwünscht insbesondere im Hinblick auf ihre mögliche Toxizität und die damit verbundenen gesundheitlichen Risiken beim Herstellungsprozeß.

Die vorliegende Erfindung betrifft ein Verfahren zur Herstellung von Benzylidenverbindungen der allgemeinen Formel (I)

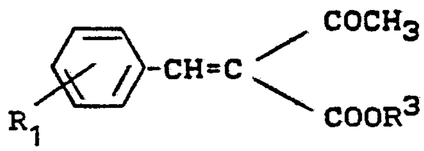

(I)

in welcher

$R^1$ für Wasserstoff oder ein oder zwei Substituenten aus der Gruppe Nitro, Cyano, Halogen, $SO_3H$, Alkyl, Alkoxy und fluoriertes Alkyl mit jeweils 1 bis 4 C-Atomen und zwei oder drei Fluorsubstituenten steht und

$R^3$ für Alkyl mit 1 bis 10 C-Atomen steht, welches gegebenenfalls durch ein Sauerstoff in der Kette unterbrochen ist oder gegebenenfalls substituiert ist durch Fluor, Chlor, Hydroxy oder eine Methylbenzylaminogruppe,

dadurch gekennzeichnet, daß man Acetale der allgemeinen Formel (II)

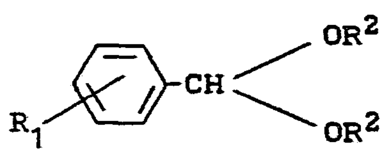

(II)

in welcher

$R^2$ jeweils für Alkyl mit 1 bis 6 C-Atomen, welches gegebenenfalls durch Phenyl substituiert ist oder die beiden Reste $R^2$ gemeinsam für einen Alkylrest mit 1 - 4 C-Atomen stehen,

mit β-Ketocarbonsäureestern der allgemeinen Formel (III)

$$CH_3CO-CH_2-COOR^3 \qquad (III)$$

in welcher

$R^3$ die oben angegebene Bedeutung hat

in Gegenwart einer Säure und gegebenenfalls in Gegenwart eines organischen Lösungsmittels bei Temperaturen zwischen 40 und 120°C und gegebenenfalls in Gegenwart katalytischer Mengen eines Amins kondensiert.

Als katalytische Mengen des Amins seien vorzugsweise 0,001 bis 0,30, insbesondere 0,01 - 0,10 Mol pro Mol Acetal genannt.

Bei Kenntnis des Standes der Technik war nicht zu erwarten, daß sich die Benzylidenverbindungen der Formel (I) direkt aus den Acetalen der allgemeinen Formel (II), ohne Isolierung der entsprechenden Aldehyde, in so hoher Reinheit und guten Ausbeuten herstellen lassen.

Die erfindungsgemäß verwendbaren Acetale der allgemeinen Formel (II) sind bekannt oder können nach bekannten Methoden hergestellt werden (vgl. DE-OS 3 212 069).

Als Säuren werden vorzugsweise verwendet: organische Mono- und Dicarbonsäuren wie z.B. Ameisensäure, Essigsäure, Propionsäure, Oxalsäure, Malonsäure, Trifluoressigsäure und Milchsäure.

Als inerte organische Lösungsmittel werden vorzugsweise verwendet: niedere Alkohole mit bis zu 10 Kohlenstoffatomen wie z.B. Methanol, Ethanol, Propanol, Isopropanol, Butanol, Hexanol, Glykol, Diglykol, Triglykol; Kohlenwasserstoffe mit bis zu 10 C-Atomen wie z.B. Hexan, Benzol, Toluol, Xylol, Ether wie z.B. Methoxy-, Ethoxy- und Butoxyethanol, 1,2-Dimethoxyethan, Glykoldimethylether, Diglykoldimethylether und Anisol.

Als katalytisch wirksame Amine seien vorzugsweise genannt: Piperidin, Pyrimidin, Pyridin, Pyridazin, Piperazin, Morpholin.

Die Durchführung des erfindungsgemäßen Verfahrens erfolgt vorzugsweise bei Temperaturen zwischen 40 bis 120, insbesondere zwischen 60 und 110°C.

Das erfindungsgemäße Verfahren sei durch folgende Beispiele erläutert.

## Ausführungsbeispiele

### Beispiel 1

98,5 Gewichtsteile o-Nitrobenzaldehyd-dimethylacetal und 63,8 Gewichtsteile Acetessigsäuremethylester sowie 10 Volumenteile Piperidin werden in 325 Volumenteilen Eisessig gelöst und über Nacht auf 80°C erwärmt. Die flüchtigen Anteile werden abgezogen und der Rückstand mit Isopropanol aufgenommen. Dabei kristallisiert nach kurzer Zeit 2-(2'-Nitrobenzyliden)-acetessigsäuremethylester aus (Fp. 97-99°C). Die Ausbeute beträgt 86 % der Theorie.

### Beispiel 2

Durchführung wie in Beispiel 1, jedoch mit Ameisensäure statt Eisessig. Die Ausbeute beträgt 84 %.

### Beispiel 3

Durchführung wie in Beispiel 1, jedoch mit Propionsäure statt Eisessig bei 110°C. Die Ausbeute beträgt 37 %.

### Beispiel 4

98,5 Gewichtsteile o-Nitrobenzaldehyd-dimethylacetal und 63,8 Gewichtsteile Acetessigsäuremethylester sowie

2 Volumenteile Piperidin werden in 163 Volumenteilen Toluol und 163 Volumenteilen Ameisensäure gelöst und über Nacht auf 80°C erwärmt. Die flüchtigen Anteile werden abgezogen und der Rückstand wird wie in Beispiel 1 behandelt. Die Ausbeute beträgt 83 %.

Beispiel 5

Durchführung wie in Beispiel 4, jedoch anstelle von Toluol/Ameisensäure wird Isopropanol/Ameisensäure verwendet und bei Rückflußtemperatur gehalten. Die Ausbeute beträgt 61 %.

Beispiel 6

98.5 Gewichtsteile o-Nitrobenzaldehyd-dimethylacetal und 63.8 Gewichtsteile Acetessigsäuremethylester sowie 10 Volumenteile Piperidin werden in 263 Volumenteilen Toluol und 50 Gewichtsteilen Chloressigsäure gelöst und über Nacht auf 80°C erwärmt. Die flüchtigen Anteile werden abgezogen und der Rückstand wie in Beispiel 1 behandelt. Die Ausbeute beträgt 33 %.

Beispiel 7

Durchführung wie in Beispiel 6, jedoch anstelle von Chloressigsäure werden 50 Gewichtsteile Malonsäure eingesetzt. Die Ausbeute beträgt 24 %.

## Patentansprüche

1. Verfahren zur Herstellung von Benzylidenverbindungen der allgemeinen Formel (I)

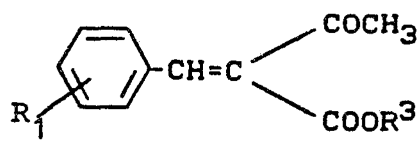

(I)

in welcher

$R^1$ für Wasserstoff oder ein oder zwei Substituenten aus der Gruppe Nitro, Cyano, Halogen, $SO_3H$, Alkyl, Alkoxy und fluoriertes Alkyl mit jeweils 1 bis 4 C-Atomen und zwei oder drei Fluorsubstituenten steht und

$R^3$ für Alkyl mit 1 bis 10 C-Atomen steht, welches gegebenenfalls durch ein Sauerstoff in der Kette unterbrochen ist oder gegebenenfalls substituiert ist durch Fluor, Chlor, Hydroxy oder eine Methyl- benzylaminogruppe,

dadurch gekennzeichnet, daß man Acetale der allgemeinen Formel (II)

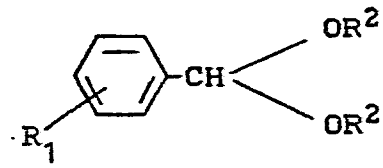

(II)

in welcher

$R^2$ jeweils für Alkyl mit 1 bis 6 C-Atomen, welches gegebenenfalls durch Phenyl substituiert ist oder die beiden Reste $R^2$ gemeinsam für einen Alkylrest mit 1 - 4 C-Atomen stehen,

mit β-Ketocarbonsäureestern der allgemeinen Formel (III)

$$CH_3CO\text{-}CH_2\text{-}COOR^3 \quad (III)$$

in welcher

$R^3$ die oben angegebene Bedeutung hat

in Gegenwart einer Säure und gegebenenfalls in Gegenwart eines organischen Lösungsmittels bei Temperaturen zwischen 40 und 120°C und gegebenenfalls in Gegenwart katalytischer Mengen eines Amins kondensiert.

2. Verfahren gemäß Anspruch 1, dadurch gekennzeichnet, daß man als Säuren organische Carbonsäuren einsetzt.

3. Verfahren gemäß Anspruch 1, dadurch gekennzeichnet, daß man als Katalysator ein Amin aus der Gruppe Piperidin, Pyrimidin, Pyridin, Pyridazin, Piperazin oder Morpholin, oder Gemische derselben einsetzt.

4. Verfahren gemäß Anspruch 1, dadurch gekennzeichnet, daß man als inerte organische Lösungsmittel niedere Alkohole, Kohlenwasserstoffe oder Ether mit bis zu 10 Kohlenstoffatomen einsetzt.

5. Verfahren gemäß den Ansprüchen 1 bis 4, dadurch gekennzeichnet, daß man die Reaktion bei Temperaturen zwischen 60 und 110°C durchführt.

6. Verfahren gemäß Ansprüchen 1 bis 5 dadurch gekennzeichnet, daß man den Amin Katalysator in Mengen von 0,001 bis 0,30 Mol pro Mol Acetal einsetzt.